(19) Europäisches Patentamt

European Patent Office

Office européen des brevets

(11) Publication number: **0 503 583 A1**

## (12) EUROPEAN PATENT APPLICATION

(21) Application number: **92104150.5**

(22) Date of filing: **11.03.92**

(51) Int. Cl.⁵: **A61K  47/36**, A61K 47/42,
A61K 37/02

(30) Priority: **12.03.91 JP 46735/91**
**10.07.91 JP 170205/91**

(43) Date of publication of application:
**16.09.92 Bulletin  92/38**

(84) Designated Contracting States:
**AT BE CH DE DK ES FR GB GR IT LI LU MC
NL PT SE**

(71) Applicant: **TAKEDA CHEMICAL INDUSTRIES,
LTD.**
**1-1, Doshomachi 4-chome
Chuo-ku, Osaka 541(JP)**

(72) Inventor: **Igari, Yasutaka**
**3-D, 9-29 Uozakinakamachi 4-chome
Higashinada-ku, Kobe(JP)**
Inventor: **Yamada, Minoru**
**1-8, Yuyamadai 1-chome
Kawanishi, Hyogo 666-01(JP)**
Inventor: **Ogawa, Yasuaki**
**77-42, Koaza-tanita, Aza-Ohyamazaki
Ohyamazaki-cho, Otokuni-gun, Kyoto 618(JP)**

(74) Representative: **von Kreisler, Alek,
Dipl.-Chem. et al
Patentanwälte Von Kreisler-Selting-Werner,
Deichmannhaus am Hauptbahnhof
W-5000 Köln 1(DE)**

(54) Composition for sustained release of erythropoietin.

(57) A composition which comprises erythropoietin and hyaluronic acid shows a sustained-release of the medicine in a living body.

FIELD OF THE INVENTION

The present invention relates to a composition for sustained-release of erythropoietin.

BACKGROUND OF THE INVENTION

Erythropoietin is known to act on erythroblastic progenitor cells in bone marrow to promote their differentiation into red blood cells. However, this substance has been made available only by the process of extracting and purifying it from human urine, thus preventing its clinical application on a large scale. With rapid progresses in recombinant DNA technology in recent years, it became possible to mass-produce erythropoietin analogs which are similar to the natural erythropoietin of human origin. These substances contributed dramatically to the improvement of renal anemic symptoms in patients with those diseases which are suspected to be chiefly associated with compromised erythropoietin production, and a further expansion of the clinical utility of erythropoietin is foreseen.

Today, erythropoietin is clinically administered to patients by the intravenous route but since the excretion of this substance after administration is fairly rapid, it must be administered as often as twice to three times a week. Moreover, the elevation of hemoglobin concentration and of hematocrit level is preferably gradual and any abrupt rises in these parameters would increase adverse reactions such as hypertension. Thus, side effect is a serious concern if a high blood concentration is reached immediately after administration, as is inevitable in the case of intravenous injection. Therefore, some proposals have been made for overcoming this disadvantage accompanying the administration of physiologically active peptides.

Long-acting preparations of a peptide drug are generally designed to maintain a sustained blood level of the peptide in humoral fluid but according to the mechanism of development of pharmacologic effects involved, drugs of this kind may generally be classified into two categories. The first category (a) is such that the pharmacologic effect of the drug is not very dependent on effective humoral concentration and an excess of pharmacologic effect is not so detrimental to the recipient's physiology. The second category (b) is such that the pharmacologic effect of the drug is dependent on effective humoral concentration and because the abrupt onset of its pharmacologic effect or an excessive pharmacologic effect is harmful to the body, the dosage must be adjusted from time to time.

Because of its dramatic pharmacologic effect, erythropoietin requires a caution in its use so that hypertension due to more than necessary hematopoiesis should be prevented. In this sense, erythropoietin is a peptide drug belonging to category (b), which calls for frequent (for example, once in about a week) efficacy evaluation and dosage adjustment.

Meanwhile, since the activity of erythropoietin is dependent on its three-dimensional structure, it is essential, in the pharmaceutical manufacturing stage, to avoid formulations which might affect the spatial configuration of the peptide.

Therefore, should a long-acting preparation be developed that would insure a sustained efficacy of erythropoietin for a period of time corresponding to an interval of efficacy assessment (for example once in about a week), the current administration frequency of 3 times a week would be reduced to benefit the patient a great deal. This benefit not only should accrue to patients with renal anemia but also would be remarkable for patients in the field of surgery involving autologous blood transfusions where both the administration frequency and the dosage level could be decreased with great rewards. Moreover, it should be possible to minimize the abrupt increase in drug concentration immediately following administration and thereby suppress the excess reactions due to a precipitating onset of the pharmacologic action.

On the other hand, hyaluronic acid is a naturally-occurring acid mucopolysaccharide and has been used as an ethical drug such as an articular function-improving agent or an adjunct in ophthalmic surgery. It is known that an aqueous solution of hyaluronic acid has a high viscosity which generally retards diffusion of other substances.

In Japanese Patent Application Laid-open No. 62-129226 (1987) which corresponds to European Patent Publication No. 224,987, it is disclosed that a solution of hyaluronan including hyaluronic acid, its sodium salt, or hylan can release dissolved or dispersed pharmacologically active substances sustainedly due mainly to the viscosity of the solution of hyaluronan. In addition, those pharmacologically active substances that have cationic residues are diffused more slowly owing to an ionic exchange between the cationic residues and the carboxylic acid of hyaluronan. In the description of one of the working examples, tritium-labeled serotonin mixed with 0.1% aqueous solution of hyaluronic acid was put into an semi-permeable dialysis membrane bag (molecular weight cut off = 10,000) and dialyzed against distilled water. The release rate of the tritium-labeled serotonin from the dialysis bag was reduced some ten-fold compared with the comparative example where no hyaluronic acid was employed.

By utilizing the above mentioned property, Japanese Patent Application Laid-open No. 1-287041 (1989) teaches a controlled release system containing hyaluronic acid or its pharmaceutically acceptable salt and, as particularly suitable physiologically active peptides, mentions insulin, crystalline insulin zinc, amorphous insulin zinc and glucagon. In the description of working examples, the above patent literature mentions as follows. When hyaluronic acid (molecular weight 1,400,000, viscosity method) was added to a neutralized injectable solution of swine insulin at a final concentration of 1% and the resulting composition was administered subcutaneously to normal male rabbits, an overt prolongation of hypoglycemia was found as compared with a positive control group of rabbits treated with insulin alone. Thus, this depressed blood glucose level was sustained at least till 12 hours following administration and, then, this effect had disappeared by 24 hours. The same literature contains a similar example for glucagon as well and mentions that the blood glucose resumed at latest by 8 hours after administration.

Japanese Patent Application Laid-open No. 2-213 (1990) also discloses a sustained release system for biologically active peptides which incorporates hyaluronic acid or its nontoxic salt. When, in working examples, a sustained release preparation of calcitonin or elcatonin containing 5% sodium hyaluronate was administered subcutaneouly to male rats, the depression of blood calcium persisted for a minimum of 12 hours. Similarly, when a sustained release system for human growth hormone containing 5% of sodium hyaluronate was administered to male rats, the blood human growth hormone level was sustained for at least 12 hours. In either case, prolongation of blood concentration was evident as compared with the corresponding comparative example in which sodium hyaluronate was not employed.

However, none of the above inventions allude to erythropoietin, whether by way of general description or as a working example.

These published inventions invariably utilize the phenomenon of delayed diffusion of ingredients in solutions of hyaluronic acid at the administration site, and Japanese Patent Application Laid-open No. 2-213 (1990) mentions that the most preferred concentration of hyaluronic acid itself is 3 to 7%. However, because of the high viscosity of the hyaluronic acid solution, removal of the air foam represents a serious technical challenge, calling for evacuation by centrifuging or decompression. Furthermore, also because of the high viscosity, it is necessary to employ a large-gauge needle for injection which gives the patient a pain which cannot be disregarded. Japanese Patent Application Laid-open No. 1-287041 (1989) is reticent about the concentration of hyaluronic acid used but contains working examples employing 1% hyaluronic acid. However, the precautions of the package insert for Artz (Kaken Pharmaceutical), which is a 1% sodium hyaluronate preparation for articular injection, recommend the use of a comparatively large-gauge needle of about 18 to 20 G. Therefore, the pain which these preparations for subcutaneous injection give to the patient is considerable.

Furthermore, Japanese Patent Application Laid-Open No. 3-4790 (1991) discloses an aqueous system that comprises polysaccharides, proteinases, and protein-like substances. It is disclosed that the system stabilizes the proteinases and prevent the loss of enzyme activity during storage especially at high temperature. In working examples, the combination of hyaluronic acid and either bovine serum albumin or gelatin prevented the loss of esperase(Novo) activity better than the corresponding comparative examples in which either hyaluronic acid alone or protein alone was employed. However, this technique never alludes to sustained release of ethical drugs, especially erythropoietin, from the system when administered to living bodies.

Accordingly, there is a desire in the art to provide a long-acting erythropoietin composition which insures sufficient efficacy at a dosing interval of about one week through prolongation of drug release and which is lenient on the patient in terms of the pain associated with administration.

SUMMARY OF THE INVENTION

The intensive research of the present inventors for solving the above-mentioned problems revealed that when erythropoietin is mixed with hyaluronic acid, a high molecular weight compound which is known to be biodegradable and pharmacologically injectable, with the concentration of hyaluronic acid being controlled below the usual concentration, and the resulting pharmaceutical composition is administered by injection, the pharmacological efficacy of erythropoietin is sustained over a long time period without interfering with the pharmacological activity of the drug substance and, at the same time, the abrupt onset of the pharmacological effect of the drug in an early stage after administration is successfully controlled. It was also found that the above pharmaceutical composition can be easily administered using a small-gauge needle of, for example, 26 G which does not cause an appreciable pain to the patient.

The present invention provides (1) a composition for sustained release of erythropoietin, which

3

comprises erythropoietin, hyaluronic acid or its nontoxic salt and a pharmaceutically acceptable carrier, diluent or excipient, and

(2) a method for producing a composition for sustained release of erythropoietin, which comprises mixing erythropoietin with hyaluronic acid or its nontoxic salt and a pharmaceutically acceptable carrier, diluent or excipient.

BRIEF DESCRIPTION OF THE DRAWINGS

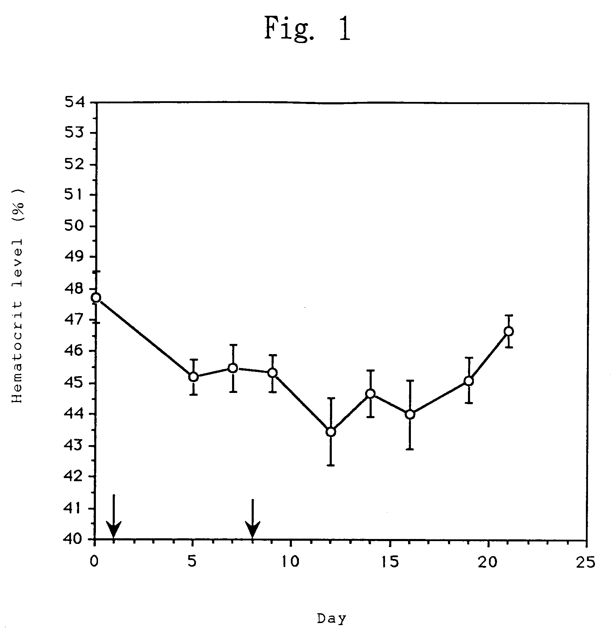

Figure 1 is a diagrammatic representation, obtained in Experimental Example 1, of the time course of hematocrit level which prevailed when Comparative Composition 1 (physiological saline) was administered twice at an interval of one week.

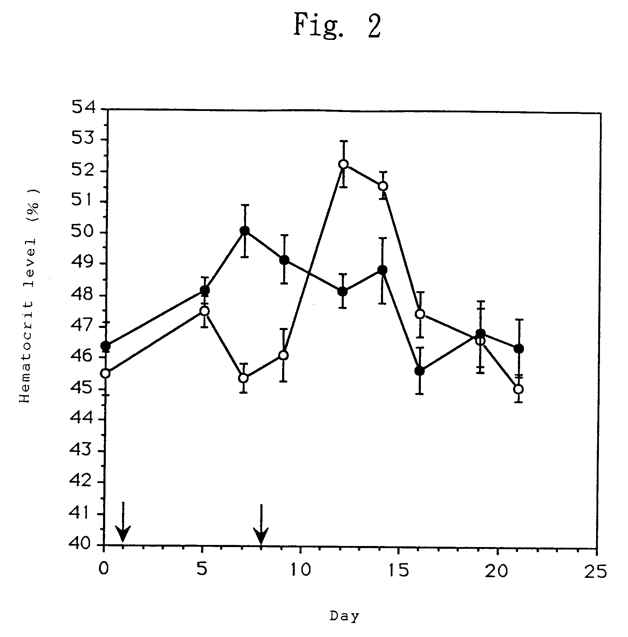

Figure 2 is a diagrammatic representation, obtained in Experimental Example 1, of the time course (open circles) of hematocrit level which prevailed when the erythropoietin injection (Comparative Composition 2) was administered twice at an interval of one week and the time course (solid circles) of hematocrit level which prevailed when the erythropoietin injection (Example 1 (1)(b)) was administered twice at an interval of one week.

In these representations, each graph represents the mean results for 4 rats at the minimum. Each plot represents the mean result of each group and the bar represents the standard error (S.E.).

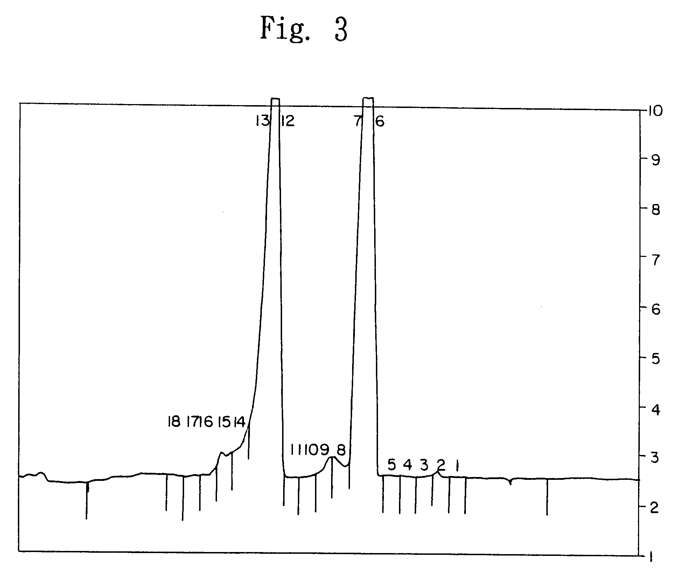

Figure 3 is a diagrammatic representation of the chromatogram (Example 2) obtained by weak anion exchange chromatography indicating the separation of human serum albumin and epoetin beta.

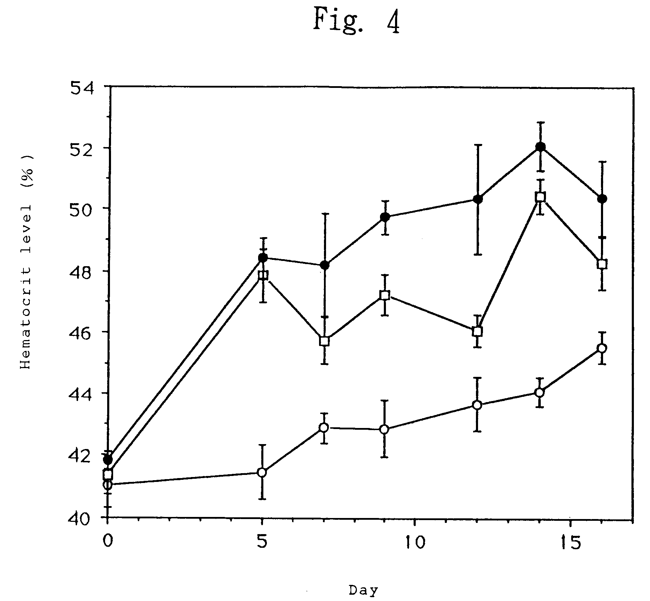

Figure 4 is a diagrammatic representation, obtained in Experimental Example 2, of the time course (solid circles) of hematocrit level which prevailed when the erythropoietin injection (Example 2) was administered twice at an interval of one week and the time course (open squares) of hematocrit level which prevailed when the erythropoietin injection (Comparative Composition 3) was administered twice at an interval of one week. The time course (open circles) of hematocrit level which prevailed when physiological saline for injection (Comparative Composition 4) was administered is shown as well.

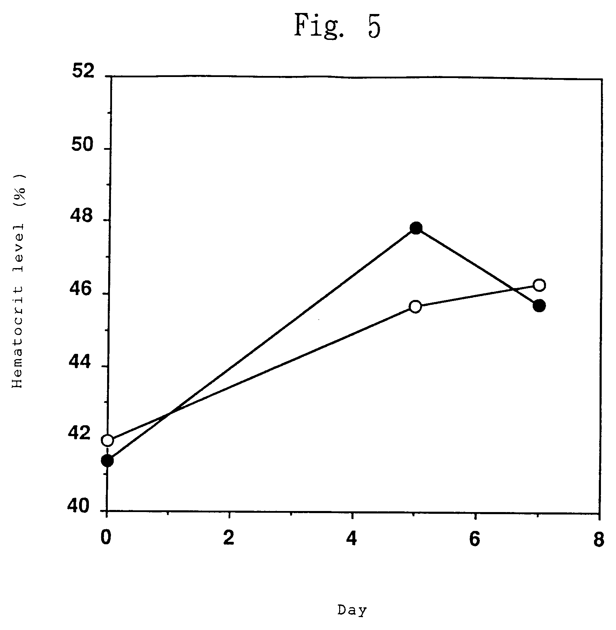

Figure 5 is a diagrammatic representation, obtained in Experimental Example 3, of the time course (open circles) of hematocrit level which prevailed when the erythropoietin injection (Comparative Composition 5) was administered and the time course (solid circles) of hematocrit level which prevailed when the erythropoietin injection (Example 3) was administered.

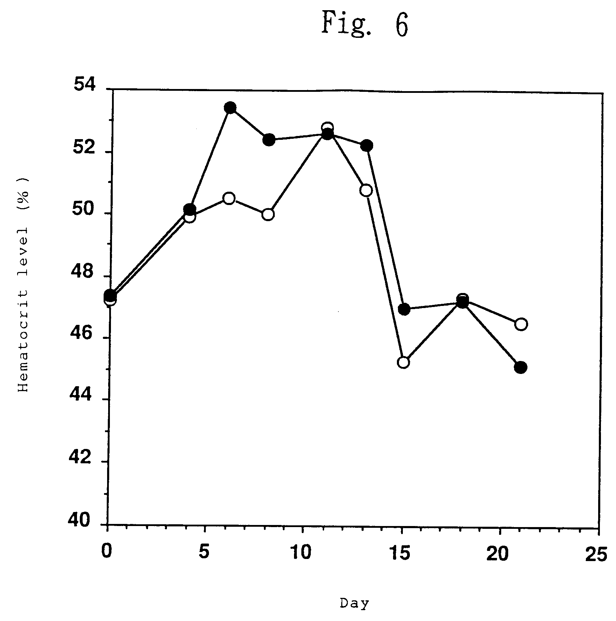

Figure 6 is a diagrammatic representation, obtained in Experimental Example 4, of the time course (open circles) of hematocrit level which prevailed when the erythropoietin injection (Comparative Composition 7) was administered and the time course (solid circles) of hematocrit level which prevailed when the erythropoietin injection (Example 5) was administered twice one week apart, respectively.

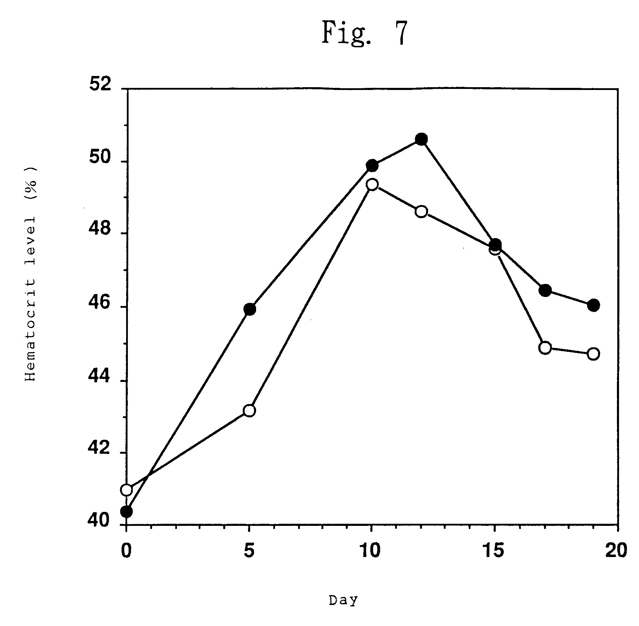

Figure 7 is a diagrammatic representation, obtained in Experimental Example 5, of the time course (open circles) of hematocrit level which prevailed when the erythropoietin injection (Comparative Composition 9) was administered and the time course (solid circles) of the hematocrit level which prevailed when the erythropoietin injection (Example 5) was administered twice one week apart, respectively.

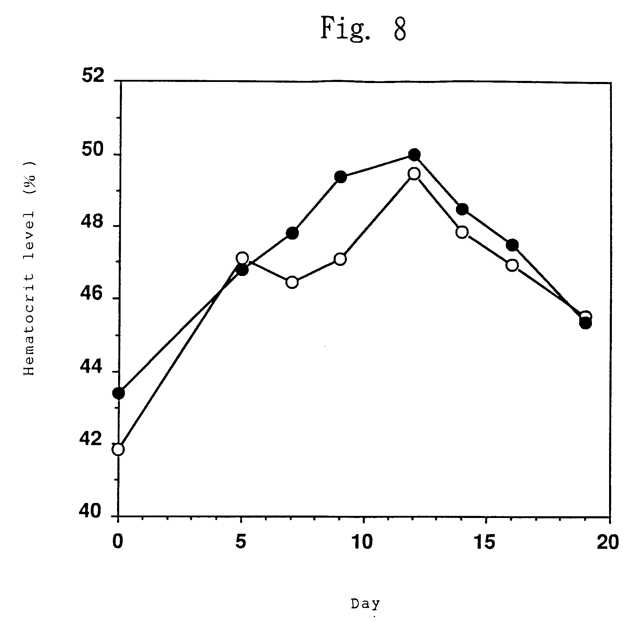

Figure 8 is a diagrammatic representation, obtained in Experimental Example 6, of the time course (open circles) of hematocrit level which prevailed when the erythropoietin injection (Comparative Composition 11) was administered and the time course (solid circles) of hematocrit level which prevailed when the erythropoietin injection (Example 6) was administered twice one week apart, respectively.

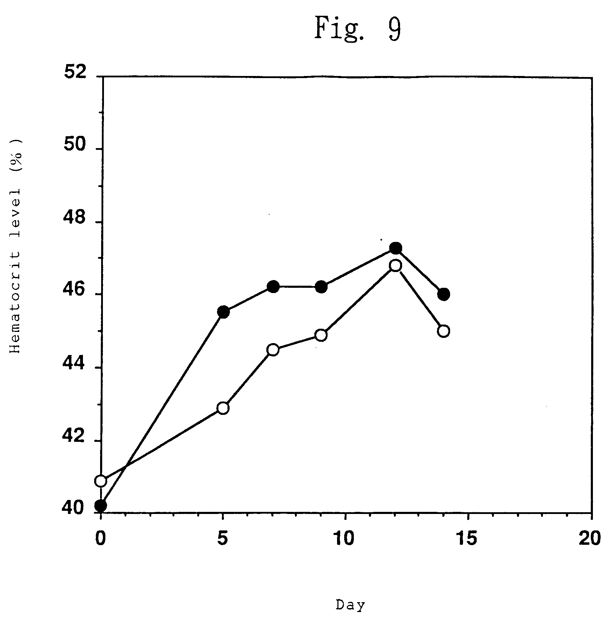

Figure 9 is a diagrammatic representation, obtained in Experimental Example 7, of the time course (open circles) of hematocrit level which prevailed when the erythropoietin injection (Comparative Composition 13) was administered and the time course (solid circles) of hematocrit level which prevailed when the erythropoietin injection (Example 7) was administered twice one week apart, respectively.

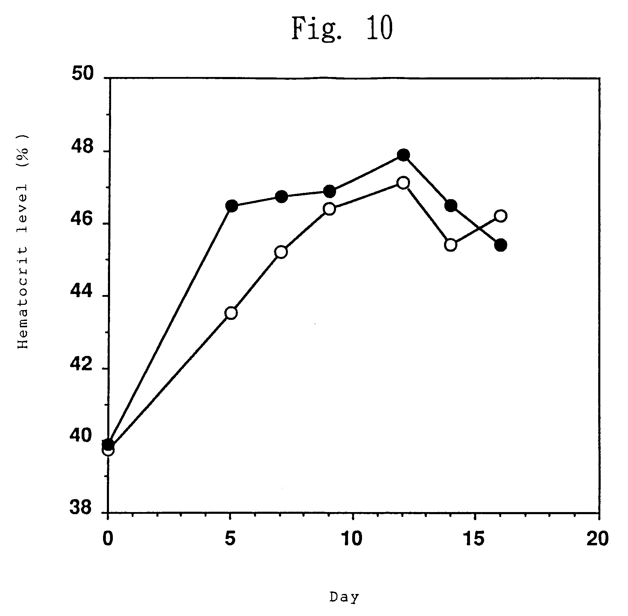

Figure 10 is a diagrammatic representation, obtained in Experimental Example 8, of the time course (open circles) of hematocrit level which prevailed when the erythropoietin injection (Comparative Composition 15) was administered and the time course (solid circles) of hematocrit level which prevailed when the erythropoietin (Example 9) was administered twice one week apart, respectively.

DETAILED DESCRIPTION OF THE INVENTION

In the context of the present invention, erythropoietin means a naturally occurring or recombinant erythropoietin as a hematopoietic glycoprotein, an erythropoietin differing in sugar chain but having hematopoietic activity, an erythropoietin lacking in sugar chain, a mutein different from them in partial amino acid sequence, or a derivative of any of them which has the same activity as erythropoietin, or an active fragment of any of said substances.

4

Examples of a naturally occuring erythropoietin include those reported by Miyake et al. (J. Biol. Chem., 252, 5558-5564 (1977)) and Recny et al. (J. Biol. Chem., 262, 17156-17161 (1987)). Examples of a recombinant erythropoietin include those reported by Jacobs et al. (Nature, 313, 806-810 (1985)), Lin et al. (Proc. Natl. Acad. Sci. U.S.A., 82, 7580-7585 (1985)), and Recny et al. (J. Biol. Chem., 262, 17156-17161 (1987)). An example of an erythropoietin differing in sugar chain includes that reported by Takeuchi et al. (Proc. Natl. Acad. Sci. U.S.A., 86, 7819-7822 (1989). An example of an erythropoietin partially lacking in sugar chain includes that reported by Dordal et al. (Endocrinology, 116, 2293-2299 (1985). An example of a derivative of an erythropoietin includes that reported by Satake et al. (Biochim. Biophys. Acta P, 1038, 125-129 (1990)), in which the charges of the amino acid residues of interest are altered (e.g. amidination, guanidination). In addition, a modification of erythropoietin by conjugating polyethylene glycol can be obtained by the well-known method (Miyata et al., Agri. Biol. Chem., 52, 1575-1581 (1988)).

Hyaluronic acid, which is employed in the present invention, is a mucopolysaccharide consisting of N-acetylglucosamine and glucuronic acid. A nontoxic salt of hyaluronic acid may also be likewise employed. The nontoxic salt includes the salts with alkali metals such as sodium, potassium, etc. and those with alkaline earth metals such as magnesium, calcium and the like. The most preferred salt is the sodium salt for purposes of the invention. Hyaluronic acid or its nontoxic salt preferably have a molecular weight of about $2 \times 10^5$ to $5 \times 10^6$ (viscosity method), more preferably about $5 \times 10^5$ to $3 \times 10^6$, still more preferably about $7 \times 10^5$ to $2.5 \times 10^6$.

While the long-acting composition of the invention contains erythropoietin and hyaluronic acid or its nontoxic salt as mentioned above, the two ingredients are preferably present in the same dosage unit. For example, this is assured when both are dissolved or suspended in sterile water or sterilized saline within the ampule or vial. This procedure may involve mixing erythropoietin with hyaluronic acid or its salt, for example, admixing a solution of erythropoietin with a solution of hyaluronic acid or its nontoxic salt thereof or adding a powder of hyaluronic acid or its nontoxic salt to a solution of erythropoietin or the latter to the former.

The goal of admixing ingredients is preferably in that erythropoietin activity is maintained and bubble formation is minimized during the process. The ingredients are put into a vessel (for example, bottle or drum) either at the same time or in any order. The total volume of the ingredients is preferably at most three quarters, more preferably at most three fifths, further preferably at most half, still more preferably at most one third of the capacity of the vessel. The vessel is shook gently and preferably rotated about its longitudinal axis for rotary blending. The number of revolution is selected according to the combination of the following conditions: the capacity of the vessel, the total volume of the ingredients, the concentration of hyaluronic acid or its nontoxic salt, the temperature of the system, and so on. The preferred number of revolution ranges from 10 round per minute(rpm) through 1000 rpm, although this is not an exclusive range. The atmosphere in the vessel can be sterile clean air or sterile clean nitrogen gas. The resultant erythropoietin-hyaluronic acid solution can be transferred to small vials or ampules, and can be further subjected to lyophilization.

The dosing solution may also be prepared by adding sterile water or sterilized saline to a lyophylisate containing both erythropoietin and hyaluronic acid or its nontoxic salt. Such a dosage unit may contain by further mixing with the common additives such as a pH adjusting agent, local anesthetic agent, solubilizer, isotonizing agent, adsorption inhibitor and so on. Preferred additives are mannitol, sorbitol, sodium chloride, glycine, ammonium acetate, water-soluble protein which does not have any practical pharmacological effect (hereinafter it is sometimes referred to as "water-soluble protein") and so on. Among said additives, water-soluble protein is preferred. The term "pharmacological effect" is defined herein as an hematopoietic effect to cause erythropoiesis.

As the water-soluble protein, it is exemplified by the protein which dissolves in water, saline or buffers and usually have its own solubility.

Specific examples of the water-soluble protein include human serum albumin, human serum globulin, collagen, gelatin.

Examples of said pH adjusting agent include glysine, ammonium acetate, citric acid, hydrochloric acid, sodium hydroxide. Examples of said local anesthetic agent include chlorobutanol, xylocaine hydrochloride. Examples of said solubilizer include glycerol, polyethylene glycol 400. Examples of said isotonizing agent include mannitol, sorbitol, sodium chloride. Examples of said adsorption inhibitor include polyoxyethylene sorbitan monooleate (Tween 80).

The preferred final concentration of hyaluronic acid or sodium hyaluronate in the pharmaceutical composition of the invention is less than one percent from viscosity points of view, namely in terms of the ease of procedures and administration. The more specific desirable concentration range is 0.02 to less than 1% (w/v), more preferably 0.1 to less than 1 % (w/v), still more preferably 0.2 to 0.8 % (w/v). While the proportion of erythropoietin in the composition should vary with the dosage and cannot be stated in general terms, dosage of the long-acting erythropoietin composition of the present invention for adult human should vary depending on the seriousness of anaemia and ranges preferably from about 100 international units (IU) to about 60000 IU per shot, more preferably from about 1000 IU to about 10000 IU, still more preferably from about 1000 IU to 6000 IU, although this is not an exclusive range and should be left to the prescription by medical doctors. Subcutaneously, generally a large drug volume can be administered but the weight ratio of erythropoietin to hyaluronic acid is dependent on the relationship between the minimum certainly injectable volume and the maximum volume not causing pain to the patient. Although it depends on the specific activity of erythropoietin, the weight ratio of erythropoietin to hyaluronic acid or its nontoxic salt thereof is exemplified as 0.0001:1 through 10:1, preferably 0.0002:1 through 5:1, more preferably 0.0002:1 through 1:1, still more preferably 0.0002:1 through 0.1:1.

The amount of water-soluble protein in the composition of one dosage unit contain is preferably 0.05 mg through 50 mg, more preferably 0.5 mg through 20 mg, still more preferably 0.75 mg through 10 mg. The weight ratio of water soluble protein to hyaluronic acid or its nontoxic salt thereof is preferably 0.001:1 through 100:1, more preferably 0.01:1 through 10:1, still more preferably 0.1:1 through 10:1.

The pH of the pharmaceutical composition of the invention should be such that, within the range tolerable for injection, it does not adversely affect the activity of erythropoietin, alter the viscosity of the solution in any drastic way or tend to form a precipitate. Specifically, the preferred pH range of the composition is pH 4 through pH 8, preferably pH 5 through pH 8, more preferably pH 6 to pH 8.

The viscosity of the present composition, when it is prepared to be a solution, is adjusted preferably to not more than about 500 centipoise (cp), more preferably about 50 to 400 cp. The viscosity depends upon the molecular weight and concentration of hyaluronic acid or its non toxic salt used, the concentration of the water-soluble protein used, the concentration of erythropoietin used, or the concentration of isotonizing agent used, so that the viscosity is adjusted by changing the concentration(s) of the compound(s) used. The values of the viscosity corresponds to those measured by employing Cone LD in E type viscosity meter (TOKIMEC, Japan) at 25°C.

The pharmaceutical composition of the present invention is preferably supplied as a liquid formulation in which erythropoietin and hyaluronic acid or its nontoxic salt are dissolved or dispersed in sterile distilled water or sterile saline. The liquid formulation can contain said excipients such as water-soluble protein. The liquid formulation are maintained at a normal refrigeration range, preferably from about +2°C to +8°C. In addition, the pharmaceutical composition of the present invention is preferably supplied as a lyophilized formulation which are obtained by lyophilizing the solution or dispersion of erythropoietin and hyaluronic acid or its nontoxic salt. The lyophilized formulation will also have a crystalizing solute and can contain said excipients such as water-soluble protein. The lyophilized formulation are maintained at a temperature range from about -20°C to about +40°C, preferably from about -5°C to about 30°C, and more preferably from about +2°C to about +30°C.

When administering, the lyophilized composition is diluted or dissolved in a pharmaceutically acceptable injectable vehicle such as distilled water for injection, physiological saline for injection.

The pharmaceutical composition of the invention is administered parenterally, namely by injection and particularly by subcutaneous injection. However, other routes of administration, such as intramuscular or intravenous injection, may be adopted depending on intended applications. Since the viscosity of the long-acting pharmaceutical composition of the invention is comparatively low, the composition can be easily aspirated from an ampule or vial into a syringe using a 25 G or 26 G needle. The bubbles that may form during aspiration are readily liquidated as the solution is allowed to stand for a brief time within the syringe.

The efficacy of the present composition, when it is an aqueous injection, is sustainedly exhibited over a time period of not less than 24 hours or longer such as one day through one week. So, since the administration involves a solution, dosage adjustment is easily carried out, and since the efficacy is long enough the present composition can be administered at an interval conrresponding to an interval of efficacy assessment.

Thus, the present invention provides a long-acting erythropoietin composition which insures a sustained concentration of erythropoietin within a pharmacologically effective range and features a low concentration of hyaluronic acid or its salt.

Further, the present erythropoietin composition can be supplied in a prefilled syringe for self-administration, since the liquid formulation will not be subject to physical disturbances such as shaking due to its low viscous nature.

Working examples of the invention are presented below.

Example 1 Production of long-acting erythropoietin compositions

6

(1)(a) To one vial of Eposine Injection 3000 (Chugai Pharmaceutical, Japan) containing 3000 IU of erythropoietin (Epoetin Beta), 25 mg of mannitol and 1 mg of human serum albumin was added 2 ml of physiological saline for injection to provide an erythropoietin injection.

(b) To 1.8 ml of the above erythropoietin injection prepared as above (containing 2700 IU of erythropoietin) was added 0.6 ml of a 2% (w/v) physiological saline solution of sodium hyaluronate (average molecular weight 1,470,000 daltons) to provide a long-acting composition of erythropoietin (final concentration of hyaluronic acid 0.5%, w/v).

(2)(a) To one vial of Eposine Injection 3000 (Chugai Pharmaceutical) containing 3000 IU of erythropoietin (Epoetin Beta), 25 mg of mannitol and 1 mg of human serum albumin was added 2 ml of distilled water for injection to provide an erythropoietin injection.

(b) To 1.8 ml of the above erythropoietin injection prepared as above (containing 2700 IU of erythropoietin) was added 0.6 ml of a 2 % (w/v) aqueous solution of sodium hyaluronate (average molecular weight 1,470,000 daltons) to provide a long-acting composition of erythropoietin (final concentration of hyaluronic acid 0.5 %, w/v).

Example 2 A long-acting erythropoietin composition

The 7-vial equivalent of a human erythropoietin preparation containing 3000 IU human erythropoietin (Epoetin Beta), 25 mg of mannitol and 1 mg of human serum albumin per vial (Eposine Injection 3000, Chugai Pharmaceutical) was dissolved in 1 ml/vial of 25 mM Tris-acetic acid buffer (pH 7.0). Using a Superloop (Pharmacia), 7 ml of this solution was applied to a weak anion exchanger column (Mab Column, J. T. Baker, USA) connected to an Ultrochrom GTi HPLC system (Pharmacia) and elution was carried out on a linear gradient from 25 mM Tris-acetic acid buffer (pH 7.0) to 2M sodium acetate buffer (pH 6.0). (The flow rate was 0.8 ml/min. and the detection wavelength was 280 nm). The eluate was collected in 1.6 ml fractions using a Super Rack (Pharmacia). The chromatogram obtained is shown in Fig. 3. Test tubes numbered 8 to 11 were pooled and the human erythropoietin in the eluate was assayed by ELISA (EPO-ELISA; Boehringer, Mannheim). It is apparent from Fig. 3 that serum albumin appears as two broad peaks on both sides of the erythropoietin band, indicating a neat resolution from erythropoietin.

To 0.7 ml (26.9 $\mu$g) of the human erythropoietin solution thus obtained was added 4 $\mu$l of Albumin Nichiyaku (Nippon Seiyaku) containing 20% (w/v) of human serum albumin, followed by addition of 1.4 ml of physiological saline for injection (Fuso Pharmaceutical) to make 2.1 ml. To this solution was added 10.5 mg of sodium hyaluronate (average molecular weight 1,470,000 daltons, Genzyme). The viscosity value was 297 cp.

Example 3 A long-acting erythropoietin composition

A glass vial (capacity: ca. 5 ml) was filled with 0.7 ml (26.9 $\mu$g) of the human erythropoietin solution (Epoetin Beta) obtained in Example 2, followed by addition of 0.7 ml of physiological saline (Fuso Pharmaceutical). Then, 0.7 ml of a 1.5% (w/v) solution of sodium hyaluronate (average molecular weight 1,470,000 daltons, Genzyme) in physiological saline for injection was added.

The glass vial (capacity: ca. 5 ml) containing the above solutions was hermetically closed and subjected to rotary blending in a 200 ml egg-plant-type flask equipped with a three-one motor (Heydon) by rotation about its longitudinal axis for about 1 hour (20-100 rpm). The procedure gave a long-acting dosing solution substantially free of bubbles.

Example 4 A long-acting erythropoietin composition

A glass vial (capacity: ca. 5 ml) was filled with 0.7 ml (26.9 $\mu$g) of the human erythropoietin solution (Epoetin Beta) obtained in Example 2 followed by addition of 4 $\mu$l of Albumin Nichiyaku (Nippon Seiyaku) containing 20 % (w/v) of human serum albumin and 0.7 ml of physiological saline for injection (Fuso Pharmaceutical). To this mixture was added 0.7 ml of a 1.5 % (w/v) solution of sodium hyaluronate (average molecular weight 1,470,000 daltons, Genzyme) in physiological saline for injection (Fuso Pharmaceutical). The glass vial (capacity: ca. 5 ml) containing these solutions was hermetically closed and subjected to rotary blending in a 200 ml egg-plant-type flask equipped with a three-one motor (Heydon) by rotation (20-100 rpm) about its longitudinal axis for about one hour. The above procedure gave a long-acting dosing solution substantially free of bubbles.

Example 5 A long-acting erythropoietin preparation

In 2 ml of physiological saline for injection was dissolved Eposine Injection 3000 (Chugai Pharmaceutical) containing 3000 IU of erythropoietin (Epoetin Beta), 25 mg of mannitol and 2 mg of human serum albumin to provide an erythropoietin injection. A glass vial (capacity: ca. 5 ml) was filled with 1.5 ml of the above injection, followed by addition of 0.5 ml of a 2 % (w/v) solution of sodium hyaluronate (average molecular weight 1,470,000: Genzyme) in physiological saline. The glass vial (capacity: ca. 5 ml) containing these solutions was hermetically closed and subjected to rotary blending by rotation about its longitudinal axis (20-100 rpm) in a 200 ml egg-plant-type flask equipped with a three-one motor (Heydon) for about one hour. The procedure gave a long-acting dosing solution substantially free of bubbles. The above procedure was repeated to provide a required quantity of the long-acting dosing solution.

Example 6 A long-acting erythropoietin composition

In 2 ml of physiological saline for injection was dissolved Eposine Injection 3000 (Chugai Pharmaceutical) containing 3000 IU of erythropoietin (Epoetin Beta), 25 mg of mannitol and 1 mg of human serum albumin to provide an erythropoietin injection. A glass vial (capacity: ca. 5 ml) was filled with 1.7 ml of the above injection and, then, 0.19 ml of a 2% (w/v) solution of sodium hyaluronate (average molecular weight 1,470,000; Genzyme) in physiological saline was added. The glass vial (capacity: ca. 5 ml) containing these solutions was hermetically closed and subjected to rotary blending by rotation about its longitudinal axis (20-100 rpm) in a 200 ml egg-plant-type flask equipped with a three-one motor (Heydon) for about one hour. The procedure gave a long-acting dosing solution substantially free of bubbles.

Example 7 A long-acting erythropoietin composition

A glass vial (capacity: ca. 5 ml) was filled with 1.3 ml of Espo Injection (Kirin Brewery), a human erythropoietin preparation containing 3000 IU of erythropoietin (Epoetin Alpha) and 5 mg of human serum albumin, followed by addition of 1.3 ml of Artz, a sodium hyaluronate preparation (average molecular weight: ca. 800,000-900,000, Seikagaku Kogyo, Japan). The glass vial (capacity: ca. 5 ml) containing these solutions was hermetically closed and subjected to rotary blending in a 200 ml egg-plant-type flask equipped with a three-one motor (Heydon) by rotation about its longitudinal axis (20-100 rpm) for about 1 hour. The procedure gave a long-acting dosing solution substantially free of bubbles.

Example 8 A long-acting erythropoietin composition

A glass vial (capacity: ca. 5 ml) was filled with 1.4 ml of Espo Injection (Kirin Brewery), a human erythropoietin preparation containing 3000 IU of erythropoietin (Epoetin Alpha) and 5 mg of human serum albumin, followed by addition of 0.7 ml of a 1.5% (w/v) solution of sodium hyaluronate (average molecular weight: ca. 500,000) in physiological saline. The glass vial (capacity: ca. 5 ml) containing these solutions was hermetically closed and subjected to rotary blending in a 200 ml egg-plant-type flask equipped with a three-one motor (Heydon) by rotation about its longitudinal axis (20-100 rpm) for about 1 hour. The procedure gave a long-acting dosing solution substantially free of bubbles.

Example 9 A long-acting erythropoietin composition

A glass vial (capacity: ca. 5 ml) was filled with 1.4 ml of Espo Injection (Kirin Brewery), a human erythropoietin preparation containing 3000 IU of erythropoietin (Epoetin Alpha) and 5 mg of human serum albumin, followed by addition of 0.7 ml of a 1.5% (w/v) solution of sodium hyaluronate (average molecular weight: 1,470,000, Genzyme) in physiological saline. The glass vial (capacity: ca. 5 ml) containing these solutions was hermetically closed and subjected to rotary blending in a 200 ml egg-plant-type flask equipped with a three-one motor (Heydon) by rotation about its longitudinal axis (20-100 rpm) for about 1 hour. The procedure gave a long-acting dosing solution substantially free of bubbles.

Example 10 A long-acting erythropoietin composition

A glass vial (capacity: ca. 5 ml) was filled with 1.4 ml of Espo Injection (Kirin Brewery), a human erythropoietin preparation containing 3000 IU of erythropoietin (Epoetin Alpha) and 5 mg of human serum albumin, followed by addition of 0.7 ml of a 0.9% (w/v) solution of sodium hyaluronate (average molecular weight: 2,300,000) in physiological saline. The glass vial (capacity: ca. 5 ml) containing these solutions was hermetically closed and subjected to rotary blending in a 200 ml flask equipped with a three-one motor (Heydon) by rotation about its longitudinal axis (20-100 rpm) for about 2 hours. The procedure gave a long-acting dosing solution substantially free of bubbles.

Example 11 A long-acting erythropoietin composition

A glass vial (capacity: ca. 5 ml) was filled with 1.3 ml of Espo Injection (Kirin Brewery), a human erythropoietin preparation containing 3000 IU of erythropoietin (Epoetin Alpha) and 5 mg of human serum albumin, followed by addition of 1.3 ml of a 1.5% (w/v) solution of sodium hyaluronate (average molecular weight: 1,470,000, Gemzyme) in physiological saline. The glass vial (capacity: ca. 5 ml) containing these solutions was hermetically closed and subjected to rotary blending in a 200 ml flask equipped with a three-one motor (Heydon) by rotation about its longitudinal axis (20-100 rpm) for about 2 hours. The procedure gave a long-acting dosing solution substantially free of bubbles.

Experimental Example 1

Comparative Composition 1: Physiological saline for injection, 2 ml.
Comparative Composition 2: To one vial of Eposine Injection 3000 (Chugai Pharmaceutical) containing 3000 IU of erythropoietin (Epoetin Beta), 25 mg of mannitol and 1 mg of human serum albumin was added 2 ml of physiological saline for injection to provide an erythropoietin injection.

The composition Example 1(1)(b) and Comparative Composition 2 were respectively administered twice at an interval of one week, each dose corresponding to a 7-day dosage based on 135 IU of erythropoietin/kg/day, subcutaneously at the back of 8-week-old male SD rats. As a negative control, Comparative Composition 1, physiological saline for injection, was similarly administered. Before administration and at timed intervals after administration, about 0.4 ml portions of blood were drawn into microhematocrit capillary tubes (Dramont Scientific) and the hematocrit level was determined (KH120M centrifuge, Kubota).

The results are plotted in Figs. 1 and 2. The saline group showed no increase in hematocrit level but rather showed a transient decrease in hematocrit level due to blood collection (Fig. 1). With Comparative Composition 2, some elevation of hematocrit level was found 5 days after the first dose but the hematocrit level fell back on day 7 and a sharp rise of hematocrit level occurred after the second dose (Fig. 2, open circles). On the other hand, with the Composition of Example 1(1)(b), the hematocrit level rose gradually on days 5 to 7 after the first dose and maintained a substantial plateau till 2 weeks after initiation of the experiment, with the second dose intervening (Fig. 2, solid circles). This suggests that the humoral concentration of erythropoietin is sustained and controlled within the pharmacologically effective range. An abrupt elevation of hematocrit level is undesirable and any behavior of hematocrit level that is typically portrayed in Fig. 2 (open circles) is objectionable. From this point of view, too, it is clear that the Composition of Example 1(1)(b) is an excellent long-acting preparation of erythropoietin.

Experimental Example 2

Comparative Composition 3: To 0.7 ml of a solution of human erythropoietin (26.9 $\mu$g) as prepared in Example 2 was added 1.4 ml of physiological saline (Fuso Pharmaceutical) for injection to make 2.1 ml. Then, 1.5 mg of sodium hyaluronate (average molecular weight 1,470,000 daltons; Genzyme) was added to the mixture.
Comparative Composition 4: Physiological saline for injection, 2 ml.

The composition of Example 2 and Comparative Composition 3, both for injection, were respectively administered subcutaneously at the back of 8-week-old male SD rats in the 7-day equivalent dose of 1.83 $\mu$g/kg/day twice one week apart. As a control, Comparative Composition 4, i.e. physiological saline for injection, was similarly administered. Before administration and serially after administration, about 0.4 ml of blood was withdrawn (EDTA•2Na added as anticoagulant) into a microhematocrit capillary tube (Dramont Scientific) and the hematocrit level was determined (KH120M centrifuge, Kubota). The results are plotted in Fig. 4. Fig. 4 is a diagram showing the time courses of hematocrit level as found when Comparative Composition 3 (open squares), Comparative Composition 4 (open circles) and Erythropoietin Composition of Example 2 (solid circles) were administered twice one-week apart. In the group treated with Comparative Composition 4, i.e. physiological saline, the hematocrit level was not elevated but remained almost unchanged. As to Comparative Composition 3 vs. Composition of Example 2, the hematocrit level rose after the first dose in both groups. After the second dose, however, the hematocrit level remained higher up to day 14 after administration in the group treated with the composition of Example 2 than in the group treated

9

with Comparative Composition 3. The above results indicate that although an erythropoietin-hyaluronate system gives a sustained erythropoietin effect, addition of serum albumin to the same system potentiates the pharmacologic effect with the same amounts of erythropoietin and hyaluronate.

Experimental Example 3

An injection was prepared according to the following formula and tested.

Comparative Composition 5: To 0.7 ml (26.9 μg) of the human erythropoietin (Epoetin Beta) mentioned above was added 1.4 ml of physiological saline for injection (Fuso Pharmaceutical) to make 2.1 ml.

Comparative Composition 6: Physiological saline for injection, 2 ml.

The injectable composition of Example 3 and Comparative Composition 5 were respectively administered subcutaneously at the back of 8-week-old male SD rats in the 7-day equivalent dose of 0.549 μg/rat/day. As a control, Comparative Composition 6, i.e. physiological saline, was similarly administered. Before administration and serially after administration, about 0.4 ml of blood was withdrawn (anticoagulant EDTA•2Na) into a microhematocrit capillary tube (Dramont Scientific) and the hematocrit level was determined (KH120M centrifuge, Kubota).

In the group treated with Comparative Composition 6, i.e. physiological saline, the hematocrit level did not rise but remained almost unchanged. Fig. 5 shows the results of administration of the composition of Example 3 (solid circles) and Comparative Composition 5 (open circles). In the group treated with the composition of Example 3, the hematocrit level rose gradually following administration, reached a peak on day 5 and, then, declined somewhat on day 7. In the group treated with Comparative Composition 5, the hematocrit level was elevated following administration but the elevation was not as great as in the group treated with the composition of Example 3. The above results indicate that an erythropoietin-hyaluronate system insures a sustained erythropoietin effect with a minimal variation of hematocrit.

Experimental Example 4

An injection was prepared according to the following formula and tested.

Comparative Composition 7: Eposine Injection 3000 (Chugai Pharmaceutical) containing 3000 IU of erythropoietin (Epoetin Beta), 25 mg of mannitol and 1 mg of human serum albumin was dissolved in 2 ml of physiological saline for injection to provide an erythropoietin injection.

Comparative Composition 8: Physiological saline for injection, 2 ml.

The composition of Example 5 and Comparative Composition 7 were respectively administered subcutaneously at the back of 8-week-old SD rats in the 7-day equivalent dose of 121.5 IU/rat/day twice one week apart. As a control, Comparative Composition 6, i.e. physiological saline, was similarly administered. Before administration and serially after administration, about 0.4 ml of blood was withdrawn (EDTA•2Na added as anticoagulant) into a microhematocrit capillary tube (Dramont Scientific) and the hematocrit level was determined (KH120M centrifuge, Kubota).

In the saline group treated with Comparative Composition 8, the hematocrit level was not elevated but remained almost unchanged. Fig. 6 shows the time courses of hematocrit level after administration of the composition of Example 5 (solid circles) and Comparative Composition 7 (open circles). In the group given the composition of Example 5 vs. the group given Comparative Composition 7, both groups showed elevations of hematocrit level but the increase was less prominent in the latter group. After the second dose, whereas the hematocrit level in the group given the composition of Example 5 showed a substantial plateau till day 14, the group given Comparative Composition 7 showed a major upsurge in hematocrit level again. The above results suggest that an erythropoietin-hyaluronate system insures a sustained erythropoietin effect with a reduced variation in hematocrit level.

Experimental Example 5

An injection was prepared according to the following formula and tested.

Comparative Composition 9: Eposine Injection 3000 (Chugai Pharmaceutical) containing 3000 IU of erythropoietin (Epoetin Beta), 25 mg of mannitol and 1 mg of human serum albumin was dissolved in 2 ml of physiological saline for injection to provide an erythropoietin injection.

Comparative Composition 10: Physiological saline for injection, 2 ml.

The composition of Example 5 and Comparative Composition 9 were respectively administered subcutaneously at the back of 8-week-old male SD rats in the 7-day equivalent dose of 18 IU/rat/day twice one week apart. As a control, Comparative Composition 10 was similarly administered. Before administration and serially after administration, about 0.4 ml of blood was withdrawn (EDTA·2Na added as anticoagulant) into a microhematocrit capillary tube (Dramont Scientific) and the hematocrit level was determined (KH120M centrifuge, Kubota).

In the saline group treated with Comparative Composition 10, the hematocrit level was not elevated but remained almost unchanged. Fig. 7 shows the time courses of hematocrit level after administration of the composition of Example 5 (solid circles) and Comparative Composition 9 (open circles). In the group given the composition of Example 5 vs. the group given Comparative Composition 9, the hematocrit level rose after the first dose in both groups but the elevation was less pronounced in the latter group. After the second dose, the hematocrit level in the group given the composition of Example 5 rose more gradually, peaked on day 12 after administration and then declined gradually. In the group treated with Comparative Composition 9, the hematocrit level after the second dose rose rapidly, peaked on day 10 and, then, declined gradually. At all observation time points, the hematocrit level was lower in this group than in the group given the composition of Example 5. The above results indicate that an erythropoietin-hyaluronate system insures a sustained erythropoietin effect with reduced variation.

Experimental Example 6

An injection was prepared according to the following formula and tested.

Comparative Composition 11: Eposine Injection 3000 (Chugai Pharmaceutical) containing 3000 IU of erythropoietin (Epoetin Beta), 25 mg of mannitol and 1 mg of human serum albumin was dissolved in 2 ml of physiological saline for injection to provide an erythropoietin injection.

Comparative Composition 12: Physiological saline for injection, 2 ml.

The composition of Example 6 and Comparative Composition 11 were respectively administered subcutaneously at the back of 8-week-old male SD rats in the 7-day equivalent dose of 40.5 IU/rat/day twice, one week apart. As a control, Comparative Composition 12, i.e. physiological saline, was similarly administered. Before administration and serially after administration, about 0.4 ml of blood was withdrawn (EDTA·2Na added as anticoagulant) into a microhematocrit capillary tube (Dramont Scientific) and the hematocrit level was determined (KH120M centrifuge, Kubota).

In the saline group treated with Comparative Composition 12, the hematocrit level was not elevated but remained almost unchanged. Fig. 8 shows the time courses of hematocrit level after administration of the composition of Example 6 (solid circles) and Comparative Composition 11 (open circles). Both the group given the composition of Example 6 and the group given Comparative Composition 11 showed elevations of hematocrit level but the increase was less prominent in the latter group, which showed a downturn as early as day 7. After the second dose, whereas the hematocrit level in the group treated with the composition of Example 6 continued to rise gradually till day 12, the hematocrit level in the group given comparative Composition 11 increased slowly at first and sharply thereafter. The above results indicate that an erythropoietin-hyaluronate system insures a sustained erythropoietin effect with reduced variation.

Experimental Example 7

An injection was prepared according to the following formula and tested.

Comparative Composition 13: Espo Injection 3000 (Kirin Brewery), a human erythropoietin preparation containing 3000 IU of erythropoietin (Epoetin Alpha) and 5 mg of human serum albumin.

Comparative Composition 14: Physiological saline for injection, 2 ml.

The composition of Example 7 and Comparative Composition 13 were respectively administered subcutaneously at the back of 7-week-old male SD rats in the 7-day equivalent dose of 40.5 IU/rat/day twice, one week apart. As a control, Comparative Composition 14, i.e. physiological saline, was similarly administered. Before administration and serially after administration, about 0.4 ml of blood was withdrawn (EDTA·2Na added as anticoagulant) into a microhematocrit capillary tube (Dramont Scientific) and the hematocrit level was determined (KH120M centrifuge, Kubota).

In the saline group treated with Comparative Composition 14, the hematocrit level was not elevated but remained almost unchanged. Fig. 9 shows the time courses of hematocrit level after administration of the composition of Example 7 (solid circles) and Comparative Composition 13 (open circles). In the group given the composition of Example 7 vs. the group given Comparative Composition 13, both groups showed elevations of hematocrit level but the increase was less prominent in the latter group. After the second dose, whereas the hematocrit level in the group given the composition of Example 7 showed a substantial plateau till day 14, the hematocrit level in the group given Comparative Composition 13 rose slowly at first and sharply thereafter. The above results indicate that an erythropoietin-hyaluronate system insures a sustained erythropoietin effect with reduced variation.

Experimental Example 8

An injection was prepared according to the following formula and tested.

Comparative Composition 15: Espo Injection 3000 (Kirin Brewery), a human erythropoietin preparation containing 3000 IU of erythropoietin (Epoetin Alpha) and 5 mg of human serum albumin.

Comparative Composition 16: Physiological saline for injection, 2 ml.

The composition of Example 9 and Comparative Composition 15 were respectively administered subcutaneously at the back of 7-week-old male SD rats in the 7-day equivalent dose of 40.5 IU/rat/day twice, one week apart. As a control, Comparative Composition 16, i.e. physiological saline, was similarly administered. Before administration and serially after administration, about 0.4 ml of blood was withdrawn (EDTA·2Na added as anticoagulant) into a microhematocrit capillary tube (Dramont Scientific) and the hematocrit level was determined (KH120M centrifuge, Kubota).

In the saline group treated with Comparative Composition 16, the hematocrit level was not elevated but remained almost unchanged. Fig. 10 shows the time courses of hematocrit level after administration of the composition of Example 9 (solid circles) and Comparative Composition 15 (open circles). In the group given the composition of Example 9 vs. the group given Comparative Composition 15, both groups showed elevations of hematocrit level but the increase was less prominent in the latter group. After the second dose, the hematocrit level in the group given the composition of Example 9 showed a substantial plateau till day 12, and, then, declined gradually. In the group given Comparative Composition 15, however, the hematocrit level after the second dose continued to rise, peaked on day 12, then, declined. The above results indicate that an erythropoietin-hyaluronate system minimizes the variation of erythropoietin effect and, yet, insures a sustained effect.

Experimental Example 9

An injection was prepared according to the following formula and tested.

Comparative Composition 17: Espo Injection 3000 (Kirin Brewery), a human erythropoietin preparation containing 3000 IU of erythropoietin (Epoetin Alpha) and 5 mg of human serum albumin.

Comparative Composition 18: Physiological saline for injection, 2 ml.

The composition of Example 10 and Comparative Composition 17 were respectively administered subcutaneously at the back of 7-week-old male SD rats in the 7-day equivalent dose of 40.5 IU/rat/day. As a control, Comparative Composition 18, i.e. physiological saline, was similarly administered. Before administration and serially after administration, about 0.4 ml of blood was withdrawn (EDTA 2Na added as anticoagulant) into a microhematocrit capillary tube (Dramont Scientific) and the hematocrit level was determined (KH120M centrifuge, Kubota).

In the group treated with Comparative Composition 18. i.e. physiological saline, the hematocrit level was not elevated but remained nearly constant. In the group given the composition of Example 10 vs. the group given Comparative Composition 17, the hematocrit level rose after administration in both groups but the increase was less pronounced in the group treated with Comparative Composition 17.

| | Hematocrit (%) | |
| --- | --- | --- |
| | Before administration | Day 5 |
| Composition of Example 10 | 39.4 | 43.0 |
| Comparative Composition 17 | 40.3 | 41.1 |

The above results indicate that an erythropoietin-hyaluronate system insures a sustained erythropoietin effect.

Experimental Example 10

An injection was prepared according to the following formula and tested.

Comparative Composition 19: Espo Injection 3000 (Kirin Brewery), a human erythropoietin preparation containing 3000 IU of erythropoietin (Epoetin Alpha) and 5 mg of human serum albumin.

Comparative Composition 20: Physiological saline for injection, 2 ml.

The composition of Example 11 and Comparative Composition 19 were respectively administered subcutaneously at the back of 7-week-old male SD rats in the 7-day equivalent dose of 40.5 IU/rat/day. As a control, Comparative Composition 20, i.e. physiological saline, was similarly administered. Before administration and serially after administration, about 0.4 ml of blood was withdrawn (EDTA•2Na added as anticoagulant) into a microhematocrit capillary tube (Dramont Scientific) and the hematocrit level was determined (KH120M centrifuge, Kubota).

In the group treated with Comparative Composition 20. i.e. physiological saline, the hematocrit was not elevated but remained nearly constant. The hematocrit levels after administration of the composition of Example 11 and Comparative Composition 19 are shown below.

| | Hematocrit (%) | |
|---|---|---|
| | Before administration | Day 5 |
| Composition of Example 11 | 40.5 | 46.1 |
| Comparative Composition 19 | 40.3 | 41.1 |

In the group given the composition of Example 11 vs. the group given Comparative Composition 19, the hematocrit level rose in both groups but less prominently in the latter group. These results indicate that an erythropoietin-hyaluronate system insures a sustained erythropoietin effect.

## Claims

1. A sustained-release pharmaceutical composition, which comprises (1) erythropoietin, (2) an amount of hyaluronic acid or its nontoxic salt effective for the sustained-release of erythropoietin, and (3) a pharmaceutically acceptable carrier, diluent or excipient.

2. A composition in accordance with claim 1, which further comprises a water-soluble protein which has no practical pharmacological effect and is capable of being introduced into a body fluid of a warm-blooded animal.

3. A composition in accordance with claim 1, which is an injectable preparation.

4. A composition in accordance with claim 1, which is a lyophilized preparation.

5. A composition in accordance with claim 1, which is a liquid preparation.

6. A composition in accordance with claim 1, wherein said erythropoietin is an erythropoietin derivative or a physiologically active substance similar to erythropoietin in biological activity but different in amino acid composition.

7. A composition in accordance with claim 1, where said hyaluronic acid or its nontoxic salt thereof has a molecular weight of $5 \times 10^5$ to $3 \times 10^6$.

8. A composition in accordance with claim 1, wherein the weight ratio of erythropoietin to hyaluronic acid or its nontoxic salt is 0.0001:1 through 10:1.

9. A composition in accordance with claim 2, wherein the water-soluble protein is human serum albumin.

10. A composition in accordance with claim 2, wherein the weight ratio of the water-soluble protein to hyaluronic acid or its nontoxic salt is 0.001 : 1 through 100:1.

## Claims for the following Contracting States : GR, ES

1. A method for preparing sustained-release pharmaceutical composition, which comprises mixing (1) erythropoietin with (2) an amount of hyaluronic acid or its nontoxic salt effective for the sustained-release of erythropoietin and (3) a pharmaceutically acceptable carrier, diluent or excipient.

2. A method in accordance with claim 1, which further comprises mixing with a water-soluble protein which has no practical pharmacological effect and is capable of being introduced into a body fluid of a warm-blooded animal.

3. A method in accordance with claim 1, which is an injectable preparation.

4. A method in accordance with claim 1, which is a lyophilized preparation.

5. A method in accordance with claim 1, which is a liquid preparation.

6. A method in accordance with claim 1, wherein said erythropoietin is an erythropoietin derivative or a physiologically active substance similar to erythropoietin in biological activity but different in amino acid composition.

7. A method in accordance with claim 1, where said hyaluronic acid or its nontoxic salt thereof has a molecular weight of $5 \times 10^5$ to $3 \times 10^6$.

8. A method in accordance with claim 1, wherein the weight ratio of erythropoietin to hyaluronic acid or its nontoxic salt is 0.0001:1 through 10:1.

9. A method in accordance with claim 2, wherein the water-soluble protein is human serum albumin.

10. A method in accordance with claim 2, wherein the weight ratio of the water-soluble protein to hyaluronic acid or its nontoxic salt is 0.001: 1 through 100 : 1.

# Fig. 1

# Fig. 2

Fig. 3

Fig. 4

## Fig. 5

## Fig. 6

Fig. 7

Fig. 8

Fig. 9

## Fig. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | WO-A-9 009 798 (IMMUNOTHERAPEUTICS)<br>* Claims 1,7-8,14,16 *<br>--- | 1-10 | A 61 K 47/36<br>A 61 K 47/42<br>A 61 K 37/02 |
| A | WO-A-9 005 522 (P. PRISELL)<br>* Claims 1-3,5; page 2, lines 15-30 *<br>--- | 1-10 | |
| D,A | DATABASE WPIL, accession no. 90-047916 [07], Derwent Publications Ltd, London, GB; & JP-A-2 000 213 (TAIHO PHARM. K.K.) 05-01-1990<br>* Abstract *<br>--- | 1-10 | |
| P,A | WO-A-9 104 058 (NORPHARMCO)<br>* Claims 1-3,6-9,60-72 *<br>----- | 1-10 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 15-05-1992 | SCARPONI U. |

EPO FORM 1503 03.82 (P0401)